# FASCICULE DE BREVET EUROPEEN

(11) **EP 3 015 132 B1**
(45) Date de publication et mention de la délivrance du brevet: **22.11.2017**
(21) Numéro de dépôt: 15188149.7
(22) Date de dépôt: 02.10.2015
(51) Int. Cl.: A61N 1/375, A61B 5/00, A61N 1/365, A61N 1/368, A61N 1/372, A61N 1/39, A61N 1/05

(54) **STIMULATEUR IMPLANTABLE ACTIF, NOTAMMENT CAPSULE INTRACARDIAQUE AUTONOME, A DÉTECTION DE L'ACTIVITÉ DE L'OREILLETTE SANS RECUEIL D'ACTIVITÉ ÉLECTRIQUE AURICULAIRE**
AKTIVER IMPLANTIERBARER STIMULATOR, INSBESONDERE AUTONOME INTRAKARDIALE KAPSEL, ZUM ERFASSEN DER VORHOF-AKTIVITÄT OHNE ERFASSUNG DER ATRIALEN ELEKTRISCHEN AKTIVITÄT
ACTIVE IMPLANTABLE STIMULATOR, IN PARTICULAR AN AUTONOMOUS INTRACARDIAC CAPSULE, WITH DETECTION OF ATRIUM ACTIVITY WITHOUT COLLECTING ATRIAL ELECTRICAL ACTIVITY

(30) Priorité: 27.10.2014 FR 1460321
(43) Date de publication de la demande: 04.05.2016
(73) Titulaire: Sorin CRM SAS, 92140 Clamart Cedex (FR); Université de Bretagne Occidentale, 29238 Brest Cedex 3 (FR); Centre Hospitalier Régional et Universitaire de Brest, 29609 Brest Cedex (FR)
(72) Inventeur: DUMONT, Jérôme, 92320 CHATILLON (FR); AMBLARD, Amel, 92330 SCEAUX (FR); RENESTO, Fabrizio, 10013 Borgofranco d'Ivrea (TO) (IT); MANSOURATI, Jacques, 29200 BREST (FR)
(74) Mandataire: Grünecker Patent- und Rechtsanwälte PartG mbB

(56) Documents cités:
- EP-A1- 2 311 524
- EP-A1- 2 499 971
- US-A1- 2013 138 006
- US-A1- 2013 325 081

## Description

L'invention concerne les "dispositifs médicaux implantables actifs" tels que définis par la directive 90/385/CEE du 20 juin 1990 du Conseil des communautés européennes, et plus particulièrement les implants permettant de surveiller en continu le rythme cardiaque et délivrer si nécessaire au coeur des impulsions électriques de stimulation, de resynchronisation et/ou de défibrillation en cas de trouble du rythme détecté par ces dispositifs.

Elle concerne notamment, mais de manière non limitative, ceux de ces dispositifs qui se présentent sous forme d'une capsule autonome destinée à être implantée dans une cavité cardiaque, notamment dans le ventricule. Ces capsules sont dépourvues de toute liaison mécanique et électrique à un dispositif principal distant, implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless",* pour les distinguer des électrodes ou des capteurs disposés à l'extrémité distale d'une sonde (*lead*) conventionnelle, parcourue sur toute sa longueur par un ou plusieurs conducteurs reliant par voie galvanique l'électrode ou le capteur à un générateur connecté à une extrémité opposée, proximale, de la sonde.

On notera toutefois que le caractère autonome de la capsule n'est pas intrinsèquement une caractéristique nécessaire de la présente invention.

L'implantation d'une capsule *leadless* dans le ventricule droit ou gauche permet de réaliser très simplement une configuration de stimulateur "simple chambre" (stimulation d'une seule cavité, ventriculaire) : la capsule *leadless* est pourvue d'une électrode de détection/stimulation en contact avec la paroi du ventricule, qui lui permet de détecter la présence ou non d'une onde de dépolarisation spontanée du ventricule, ainsi que l'instant de survenue de cette onde (marqueur ventriculaire) et si nécessaire de délivrer une impulsion de stimulation en cas de dépolarisation spontanée absente ou tardive, de manière à provoquer la contraction du ventricule.

Toutefois, ce mode de fonctionnement est limité à une stimulation de type simple chambre ventriculaire, c'est-à-dire dans laquelle un intervalle d'échappement (IE) est programmé pour provoquer une stimulation si la durée écoulée depuis la dernière détection ou stimulation du ventricule dépasse la durée de cet intervalle, ou bien ne rien faire en cas de dépolarisation ventriculaire spontanée détectée.

Ce mode de fonctionnement présente l'inconvénient que la stimulation ventriculaire n'est pas synchronisée avec la vidange de l'oreillette, et que le rythme de stimulation ne s'adapte pas au rythme sinusal à un instant donné (rythme sinoatrial). Ceci peut être gênant en cas d'effort, car le rythme ne va pas s'accélérer en dépit des besoins physiologiques accrus. Pour l'adaptation au rythme du patient, il existe des solutions mettant en oeuvre des méthodes d'asservissement de type *rate-response,* mais celles-ci sont sous-optimales par rapport aux méthodes basées sur la détection d'une activité auriculaire, dès que le patient n'est pas incompétent chronotrope.

En l'absence de dysfonction sinusale, on peut préférer au mode simple chambre ventriculaire un mode VDD. Ce mode VDD est obtenu par programmation d'un stimulateur double chambre traditionnel, mais peut être également obtenu avec un dispositif à sonde unique portant deux électrodes atriales "flottantes" pour la détection atriale, et des électrodes de stimulation/détection bipolaire ventriculaires. En revanche, toute stimulation atriale est impossible. La détection de l'activité auriculaire, c'est-à-dire de l'instant où l'oreillette se contracte, permet de déterminer le rythme sinusal instantané du patient (à partir des intervalles RR successifs) et ainsi de calculer et appliquer un délai atrioventriculaire (DAV) fonction de ce rythme. Le stimulateur fonctionnera alors en mode VDD (stimulation du ventricule à partir des signaux recueillis à la fois sur le ventricule et sur l'oreillette).

Cette manière de procéder n'est pas envisageable avec une capsule *leadless,* qui par définition est dépourvue de sonde et ne comporte donc aucun élément situé dans la région de l'oreillette.

Le problème de l'invention est, dans un tel cas, de pouvoir détecter une activité auriculaire à partir de la seule capsule *leadless* implantée dans le ventricule droit ou gauche, sans moyen ni possibilité de recueillir un signal reflétant l'activité électrique de l'oreillette.

Le même problème peut se poser dans certaines situations avec un stimulateur conventionnel (c'est-à-dire un dispositif comprenant une sonde endocavitaire connectée à un générateur distant), par exemple en cas de rupture d'une sonde auriculaire, ou lorsque les signaux délivrés en utilisant une électrode flottante (configuration de "dipôle flottant") ne sont pas d'une qualité suffisante pour pouvoir en dériver un signal d'activité auriculaire suffisamment fiable.

Diverses propositions ont été formulées pour résoudre le problème de l'obtention d'un signal auriculaire par une capsule *leadless* endocavitaire implantée dans le ventricule droit ou gauche.

Ainsi, le US 2013/0325081 A1 décrit une capsule *leadless* dont l'une des extrémités est ancrée au fond du ventricule droit et dont l'extrémité opposée, libre, est munie d'une extension en forme de fil ou d'antenne se prolongeant jusque dans l'oreillette droite. Cette extension est en contact avec la paroi de l'oreillette droite et permet de faire de la détection/stimulation dans cette cavité, rendant possible un fonctionnement de la capsule dans des modes DDD ou DDDR. Dans une autre forme de réalisation, la capsule ne comporte pas d'extension, mais est munie à son extrémité libre, opposée au point d'ancrage, d'électrodes destinées à permettre une détection en champ lointain (*far-field*) de l'activité électrique de l'oreillette droite, avec application de filtrages spécifiques ou d'une reconnaissance morphologique des signaux électriques recueillis au niveau de la capsule par ces électrodes.

De façon comparable, le US 2013/0138006 A1 utilise des électrodes situées sur des régions de la capsule qui ne sont pas en contact avec la paroi du ventricule, afin de recueillir des signaux d'électrogramme en champ lointain. La détection de l'activité auriculaire est basée sur une comparaison entre le champ proche (*near-field*) et le champ lointain (*far-field*), l'activité auriculaire étant censée être détectée lorsque le signal *far-field* dépasse un seuil et que le signal *near-field* ne le dépasse pas. Cette double détection oreillette/ventricule à partir d'électrodes ventriculaires, uniquement basée sur les signaux électriques *far-field*/*near-field,* reste complexe à mettre en oeuvre et, en pratique, de fiabilité insuffisante pour détecter la présence ou non d'une activité auriculaire avec un degré élevé de certitude.

Le EP 2 471 447 A1 décrit une configuration dans laquelle une stimulation ventriculaire gauche produite par une capsule épicardique est synchronisée sur une stimulation auriculaire gauche, également produite par une capsule épicardique. La synchronisation s'effectue par analyse de signaux conduits par les tissus cardiaques, la capsule ventriculaire fonctionnant en esclave de la capsule auriculaire. Ce dispositif implique toutefois la présence d'une capsule auriculaire et l'implantation de capsules épicardiques en contact avec la paroi externe du myocarde, procédure beaucoup plus compliquée que la mise en place d'une capsule endocavitaire unique.

Une autre proposition encore est celle du US 2013/0123872 A1, où une unique capsule *leadless* est utilisée, mais placée dans l'oreillette. Le problème est alors de détecter une activité ventriculaire depuis l'oreillette, ce qui implique la mise en oeuvre de filtres complexes pour éviter les interférences (*cross-talk*) entre signaux ventriculaires et auriculaires.

Aucun de ces dispositifs n'apporte de solution satisfaisante au problème exposé ci-dessus, car tous sont basés sur le recueil d'une information électrique témoignant de l'activité de l'oreillette et nécessitant une électrode auriculaire.

Pour résoudre ce problème, l'invention propose de détecter l'activité auriculaire à l'aide du signal délivré par un capteur d'accélération endocardiaque (EA) incorporé à la capsule *leadless.*

En effet, la contraction auriculaire se traduit par une composante spécifique observable dans le signal EA, dite "composante EA4", dont la présence ou l'absence permet de révéler l'existence, ou non, d'une contraction des oreillettes et l'instant de sa survenue. Une fois cette composante détectée, il devient possible de déclencher un DAV, comme le ferait un stimulateur double chambre programmé en VDD.

On connait des stimulateurs utilisant une sonde munie d'un capteur EA, comme décrit par exemple dans le EP 2 189 180 A1 (ELA Medical). Ce document propose d'ajuster le DAV en prenant comme point de départ du délai l'instant de fin de l'onde EA4. La recherche de la composante EA4 est réalisée après détection d'une onde P (onde de dépolarisation auriculaire spontanée) ou après une stimulation auriculaire A. Cette configuration nécessite donc de disposer d'un marqueur de l'activité électrique auriculaire (P/A), et elle n'est de ce fait pas applicable au cas d'une capsule *leadless* dépourvue de moyens de recueil de l'activité électrique de l'oreillette.

D'autres stimulateurs utilisant une sonde munie d'un capteur EA sont décrits dans le EP 2 499 971 A1 (Sorin CRM), qui concerne une technique d'analyse du signal EA permettant d'adapter dynamiquement une thérapie en fonction de l'état du patient, et dans le EP 2 311 524 A1 (Sorin CRM), qui concerne l'optimisation concomitante du DAV et du DW en fonction des résultats de l'analyse du signal EA.

Mais aucun de ces deux documents ne concerne le problème spécifique exposé plus haut, à savoir la détection d'une activité auriculaire par un dispositif (notamment une capsule *leadless*) implanté dans le ventricule droit ou gauche, mais dépourvu de sonde auriculaire ou tout autre moyen ou possibilité de recueillir un signal reflétant l'activité électrique de l'oreillette.

Le EP 2 189 182 A1 (ELA Medical) a pour objet la détection d'une activité auriculaire à partir de la composante EA4, pour confirmer ou non la capture après une stimulation auriculaire. La détection de la composante EA4 est basée sur des analyses d'énergie dans des fenêtres glissantes, qui valident la présence d'une contraction si l'énergie dépasse un seuil donné. Pour chaque cycle cardiaque, la fenêtre de recherche de la composante EA4 est référencée par rapport à l'instant de stimulation ou de détection auriculaire, présupposant comme dans le cas précédent la connaissance d'un marqueur de l'activité électrique auriculaire (P/A). En tout état de cause, dans la mesure où il s'agit de confirmer ou non la capture après stimulation de l'oreillette, le dispositif est nécessairement muni d'une électrode auriculaire, dont l'objet est précisément de permettre cette stimulation de l'oreillette.

Aucun de ces documents ne divulgue donc la manière de détecter une activité auriculaire sans disposer au préalable d'un marqueur d'activité électrique permettant de reconnaitre et confirmer l'existence de la contraction auriculaire.

Le EP 2 092 885 A1 (ELA Médical) décrit un procédé de traitement du signal EA permettant d'en extraire les principales composantes. Ce document décrit essentiellement des méthodes d'extraction des composantes EA1 et EA2, correspondant aux deux bruits principaux du coeur, ces méthodes pouvant être transposées à la détection de la composante EA4. La détection des composantes EA1 et EA2 passe par la définition de fenêtres de recherche prédéfinies, basées sur des marqueurs électriques ventriculaires et fonction du rythme cardiaque. Mais ce document ne divulgue aucune méthode qui permettrait de définir ces fenêtres pour détecter éventuellement une composante EA4 sans marqueur de l'activité électrique auriculaire.

Pour atteindre les buts ci-dessus, la présente invention propose un dispositif comprenant, de manière en elle-même connue :
- des moyens de détection d'événements ventriculaires par analyse d'un signal EGM d'électrogramme ventriculaire ;
- des moyens de stimulation ventriculaire ;
- des moyens aptes à appliquer aux moyens de stimulation ventriculaire un délai atrioventriculaire, DAV, compté à partir d'un événement auriculaire et à l'issue duquel une stimulation ventriculaire est délivrée en l'absence de détection d'un événement ventriculaire spontané ;
- un capteur d'accélération apte à délivrer un signal d'accélération endocardiaque, EA, représentatif des contractions cycliques du myocarde ; et
- des moyens d'analyse du signal EA, aptes à reconnaitre et isoler dans le signal EA une composante EA4 correspondant au quatrième pic d'accélération endocardiaque associé à une activité auriculaire.

De façon caractéristique de l'invention, ce dispositif est dépourvu de moyens de recueil d'un signal EGM auriculaire, et il comprend en outre :
- des moyens de détermination d'une fenêtre de recherche de la composante EA4 dans le signal EA ;
- des moyens aptes à avérer ou non la présence d'une composante EA4 dans la fenêtre de recherche ; et
- des moyens aptes à appliquer aux moyens de stimulation ventriculaire :
   - ledit DAV, en présence d'une composante EA4 avérée ; ou
   - un intervalle d'échappement prédéterminé, en l'absence de composante EA4 avérée.

En ce qui concerne les moyens de détermination de la fenêtre de recherche, ceux-ci comprennent des moyens aptes :
a) en présence d'une conduction atrioventriculaire, à :
   - détecter l'instant de survenue de l'évènement ventriculaire à partir du signal EGM ventriculaire ;
   - détecter dans le signal EA des composantes EA1 et/ou EA2 correspondant au premier et/ou second pics respectifs d'accélération endocardiaque associés à l'évènement ventriculaire ; et
   - déterminer la fenêtre de recherche de la composante EA4 en fonction de la position temporelle des composantes EA1 et/ou EA2 détectées, et
b) en l'absence de conduction atrioventriculaire, à :
   - appliquer une temporisation de masquage des composantes EA1 et/ou EA2 dans le signal EA, comptée à partir d'un événement ventriculaire stimulé ; et
   - déterminer la fenêtre de recherche de la composante EA4 à l'issue de la temporisation de masquage.

Selon diverses caractéristiques subsidiaires avantageuses :
- le dispositif est une capsule intracardiaque autonome comprenant un corps tubulaire pourvu à son extrémité distale d'un organe d'ancrage apte à pénétrer dans une paroi d'un ventricule d'un patient ;
- les moyens aptes à avérer ou non la présence d'une composante EA4 dans la fenêtre de recherche comprennent des moyens aptes à évaluer le niveau d'énergie global du signal EA dans la fenêtre de recherche et à comparer ce niveau d'énergie global à un seuil prédéterminé ;
- les moyens aptes à avérer ou non la présence d'une composante EA4 dans la fenêtre de recherche comprennent des moyens d'analyse morphologique du signal EA dans la fenêtre de recherche par test du passage dans des fenêtres prédéterminées de minima locaux et/ou de maxima locaux du signal EA ;
- le dispositif comprend en outre des moyens d'estimation du rythme spontané du patient en l'absence de conduction atrioventriculaire, calculé en fonction de l'intervalle de temps séparant la détection de deux composantes EA4 avérées pour des cycles cardiaques consécutifs ;
- dans ce cas, le dispositif comprend avantageusement des moyens aptes à : calculer une fréquence de stimulation ventriculaire en fonction de la valeur de rythme spontané calculée par les moyens d'estimation ; calculer l'intervalle d'échappement en fonction de la valeur de rythme spontané calculée par les moyens d'estimation ; calculer le DAV en fonction de la valeur de rythme spontané calculée par les moyens d'estimation ; et/ou calculer la durée de la fenêtre de recherche en fonction de la valeur de rythme spontané calculée par les moyens d'estimation.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 illustre une configuration conventionnelle de stimulateur simple chambre, mettant en oeuvre une sonde endocavitaire ventriculaire reliée à un boitier de générateur.
La Figure 2 illustre une configuration de stimulation simple chambre mettant en oeuvre une unique capsule *leadless* endocavitaire implantée dans le ventricule, configuration à laquelle s'applique avantageusement la présente invention.
La Figure 3 est un chronogramme illustrant un signal typique d'accélération endocardiaque recueilli par un capteur accélérométrique intégré à la capsule *leadless* de la Figure 2.
La Figure 4 est un schéma par blocs illustrant les principales étapes du procédé de l'invention, en particulier la sélection de la méthode de recherche d'une contraction auriculaire en fonction de la présence ou non d'un bloc atrioventriculaire (AV) complet.
La Figure 5 est un organigramme exposant les principales étapes du procédé selon l'invention, tel que mises en oeuvre en l'absence de bloc AV complet.
Les Figures 6 et 7 sont des chronogrammes illustrant deux méthodes respectives possibles, en l'absence de bloc AV complet, pour la détection de la composante EA4 dans le signal EA recueilli.
La Figure 8 illustre de façon schématique le principe du procédé selon l'invention, tel que mis en oeuvre en présence d'un bloc AV complet.
La Figure 9 est un organigramme exposant les principales étapes du procédé selon l'invention, tel que mis en oeuvre en présence d'un bloc AV complet.
La Figure 10 est un chronogramme illustrant la manière dont l'intervalle d'échappement est calculé et borné, en présence d'un bloc AV complet.
La Figure 11 est une caractéristique illustrant la variation de la position de la fenêtre de recherche de la composante EA4 en fonction de la durée calculée de l'intervalle d'échappement, en présence d'un bloc AV complet.

On va maintenant décrire un exemple de réalisation du dispositif de l'invention.

En ce qui concerne ses aspects logiciels, l'invention peut être mise en oeuvre par une programmation appropriée du logiciel de commande d'un dispositif stimulateur cardiaque connu, par exemple un stimulateur de type capsule *leadless* endocavitaire.

Ces dispositifs comprennent un microprocesseur programmable couplé à des circuits pour recevoir, mettre en forme et traiter des signaux électriques recueillis par des électrodes implantées, et délivrer des impulsions de stimulation à ces électrodes. L'adaptation de ces dispositifs pour réaliser l'invention est à la portée de l'homme du métier, et elle ne sera pas décrite en détail. En particulier, les logiciels conservés en mémoire et exécutés pourront être adaptés et utilisés pour mettre en oeuvre les fonctions de l'invention qui seront décrites ci-dessous

Le procédé de l'invention est en effet mis en oeuvre par des moyens principalement logiciels, au moyen d'algorithmes appropriés exécutés par un microcontrôleur ou un processeur numérique de signal. Pour la clarté de l'exposé, les divers traitements appliqués seront décomposés et schématisés par un certain nombre de blocs fonctionnels distincts et/ou de circuits interconnectés, mais cette représentation n'a toutefois qu'un caractère illustratif, ces fonctions ou circuits comprenant des éléments communs et correspondant en pratique à une pluralité de fonctions globalement exécutées par un même logiciel.

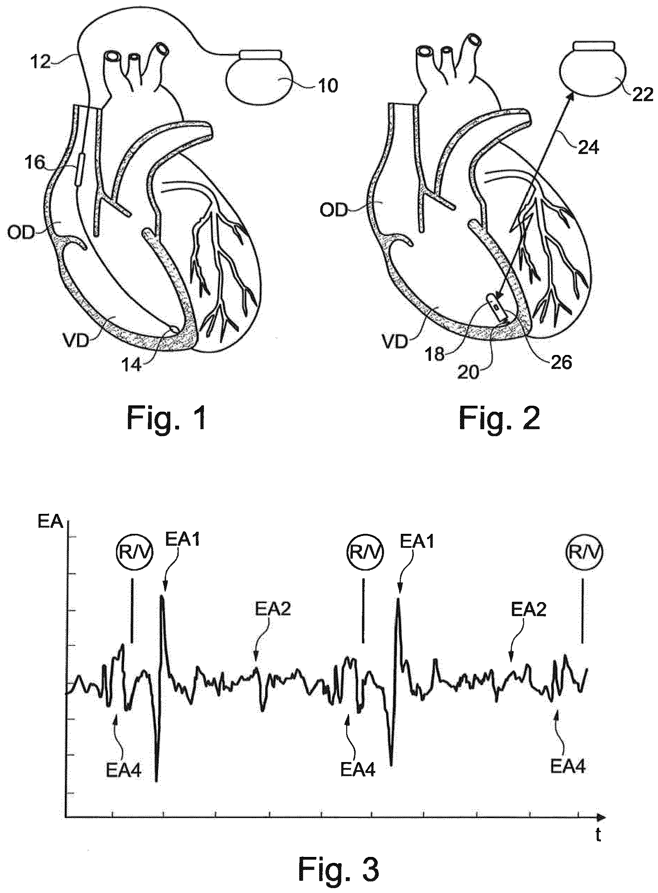

Sur la Figure 1, on a représenté une configuration conventionnelle de stimulateur simple chambre, avec un boitier de générateur 10 sur lequel est connectée une sonde endocavitaire 12 munie à son extrémité distale d'une électrode de détection/stimulation ventriculaire 14, typiquement ancrée dans l'apex du ventricule droit VD pour permettre la détection des ondes de dépolarisation spontanée de ce ventricule, ainsi que si nécessaire la stimulation de celui-ci par délivrance d'impulsions produites par le générateur 10. La sonde endocavitaire 12 est introduite via le réseau veineux et, pour atteindre le ventricule droit, traverse l'oreillette droite OD. Si l'on souhaite recueillir des signaux reflétant l'activité électrique de cette oreillette, il est possible de munir le corps de la sonde 12, dans la région traversant l'oreillette OD, d'une électrode 16 reliée au générateur 10 par un conducteur, interne à la sonde 12, distinct de celui relié à l'électrode ventriculaire 14. L'électrode auriculaire 16 et le générateur 10 forment alors un dipôle "flottant" (l'électrode 16 n'étant pas, ou pas nécessairement, en contact avec la paroi de l'oreillette) qui permet de recueillir des signaux électriques. Après filtrage et traitement, ces signaux sont susceptibles de procurer des informations sur l'activité électrique des oreillettes, avec toutefois un certain nombre de limitations en raison de la qualité parfois médiocre du signal électrique recueilli par le dipôle flottant.

La Figure 2 illustre une configuration de stimulateur mettant en oeuvre une capsule *leadless* 18 ancrée au fond du ventricule droit VD, avec une électrode 20 en contact avec la paroi du ventricule à cet endroit, cette électrode pouvant être éventuellement constituée par la vis d'ancrage de la capsule 18. La capsule *leadless* 18 est pourvue de moyens émetteurs/récepteurs lui permettant de communiquer à distance avec un boitier 22, implanté ou externe, via une liaison sans fil 24 de type radiofréquence ou HBC (*Human Body Communication,* communication par voie intracorporelle). Le boitier 22, qui joue le rôle de dispositif maitre, reçoit les signaux recueillis par la capsule *leadless* 18, analyse ces signaux et envoie éventuellement des ordres à la capsule 18 pour que celle-ci délivre des impulsions de stimulation du ventricule. Le boitier 22 peut être également utilisé en tant que passerelle avec le monde extérieur pour communiquer par télémétrie RF avec des périphériques externes tels que des programmateurs ou dispositifs de télétransmission de données.

On notera toutefois que l'invention proposée ne se limite pas à une telle configuration où une capsule *leadless* est interfacée à un boitier maitre (le boitier 22). La capsule *leadless* 18 peut en effet être une capsule fonctionnellement autonome, intégrant tous les circuits de recueil et d'analyse du signal EGM ventriculaire et de communication vers des périphériques externes.

De façon caractéristique, la capsule *leadless* 18 est munie d'un capteur 26 d'accélération endocardiaque (EA), par exemple sous la forme d'un micro-accéléromètre incorporé à la capsule 18, ce capteur étant susceptible de délivrer un signal représentatif de l'activité mécanique du myocarde. Il n'est toutefois pas indispensable que le capteur EA soit incorporé à la capsule *leadless,* l'invention étant également applicable à une analyse opérée à partir d'un signal EA délivré par un capteur accélérométrique distinct, ou même par un autre type de capteur EA implanté, tel qu'un capteur de mouvement d'une paroi du myocarde ou un capteur épicardique.

La Figure 3 illustre un exemple de signal endocardiaque EA recueilli par un tel capteur.

Sur ce chronogramme, on a indiqué les marqueurs R/V d'activité électrique ventriculaire, obtenus par analyse du signal recueilli par l'électrode 20 (marqueur R de dépolarisation spontanée) ou, en l'absence de dépolarisation spontanée, à partir de l'impulsion de stimulation délivrée sur commande du boitier 22 (marqueur V de dépolarisation stimulée).

Comme on peut le voir sur la Figure 3, le signal EA forme au cours d'un cycle cardiaque plusieurs composantes, notamment :
- une composante EA1, correspondant au premier bruit cardiaque (phonocardiogramme) et dont les variations sont étroitement liées aux variations de la pression dans le ventricule - l'amplitude du pic de la composante EA1 étant, plus précisément, corrélée au maximum positif de la variation de pression dP/dt dans le ventricule gauche ;
- une composante EA2 correspondant au second bruit cardiaque, lors de la phase de relaxation ventriculaire isovolumique produite par la décélération brusque de la masse sanguine en mouvement dans l'aorte ; et
- deux autres composantes d'amplitude très inférieure, appelées EA3 et EA4, correspondant, dans certaines conditions, aux sons S3 et S4 du phonocardiogramme.

L'invention s'intéresse plus particulièrement à la composante EA4, qui est directement liée à la présence d'une contraction auriculaire.

Cette composante EA4 présente un pic qui, comme on peut le voir sur la Figure 3, se situe immédiatement avant le pic de la composante EA1. Pour cette raison, cette composante est quelquefois désignée "EA0" par les rythmologues, car du point de vue électrique la contraction de l'oreillette précède la contraction du ventricule. En revanche, si l'on se place du point de vue du flux sanguin pompé par le myocarde, la contraction de l'oreillette (composante EA4) achève le remplissage du ventricule en fin de diastole (composante EA2) et se situe donc, du point de vue de l'hémodynamique cardiaque, après cette dernière, d'où la désignation de composante "EA4".

Le problème que résout l'invention, comme indiqué plus haut, est d'obtenir une information sur l'activité auriculaire à partir de ce seul signal EA et d'un marqueur R/V d'activité électrique ventriculaire, afin de pouvoir assurer un fonctionnement en mode VDD dès que cela est possible.

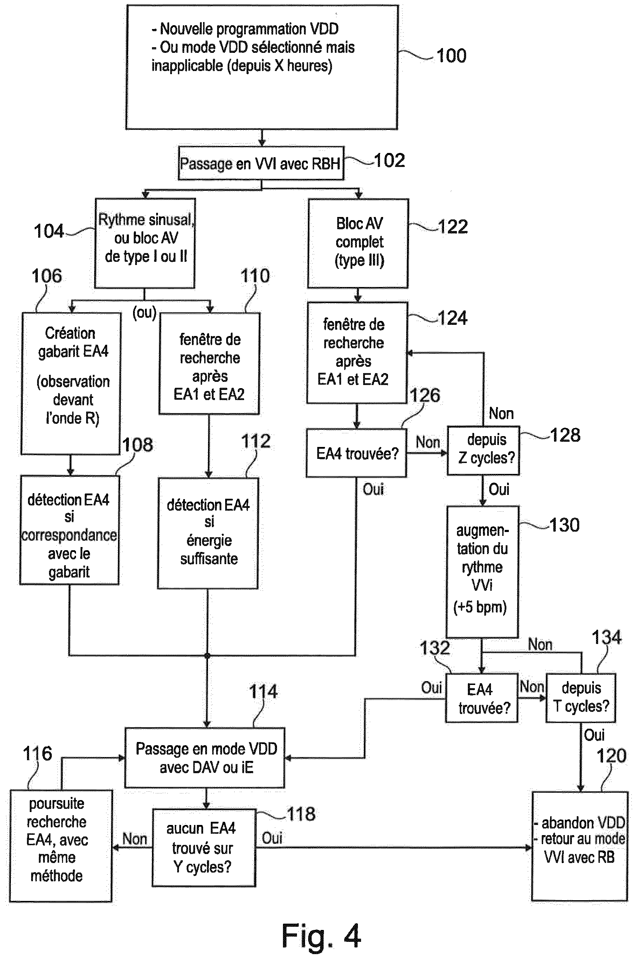

En premier lieu, comme illustré Figure 4, deux cas cliniques doivent être distingués, selon que le patient présente ou non un bloc atrioventriculaire (bloc AV) complet.

À cet effet, la capsule 18 est conçue pour pouvoir sélectionner deux rythmes de base programmés différents, à savoir :
- un rythme de base standard, ci-après RB, qui sera appliqué en cas de fonctionnement en mode VVI (c'est-à-dire en cas de synchronisation impossible avec les oreillettes), typiquement RB = 70 bpm ; ou
- un rythme de base d'hystérésis, ci-après RBH, plus faible que RB (ou égal à RB si le praticien préfère désactiver la fonctionnalité), typiquement RBH = 50 bpm. Ce rythme de base réduit correspond au rythme spontané le plus faible autorisé lorsque l'on peut se synchroniser avec les oreillettes, et a pour but de favoriser la resynchronisation avec l'oreillette même si le rythme spontané du patient est assez faible.

Le processus débute (bloc 100) sur une demande de programmation d'un mode VDD, ou bien toutes les X heures (par exemple X = 1 ou 2 heures) lorsque le mode VDD est choisi mais n'a pas pu être appliqué.

L'appareil reste alors en mode VVI mais passe temporairement à la fréquence RBH (bloc 102).

L'étape suivante (bloc 104) consiste à détecter la présence, ou non, d'un rythme spontané par recherche d'ondes R récurrentes.

Si un rythme spontané est observé, on est dans une situation correspondant à un rythme sinusal ou à un bloc AV partiel (bloc de type I ou II) n'empêchant pas complètement la conduction entre oreillette et ventricule (bloc 104).

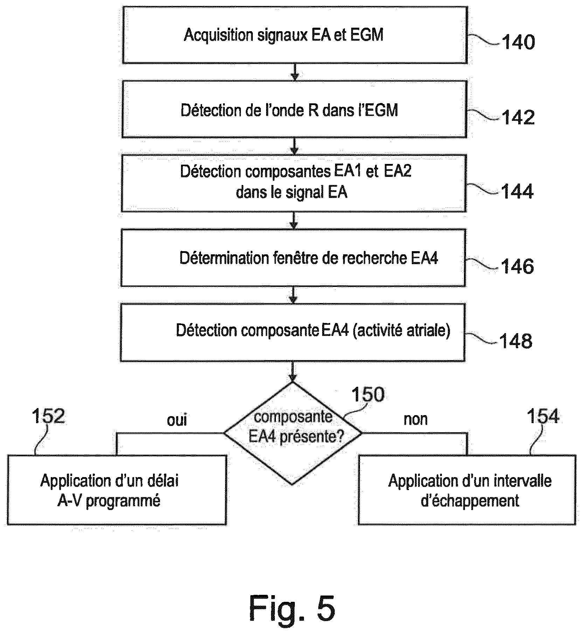

La détection de l'activité auriculaire sera alors opérée selon une première méthode, décrite en référence aux Figures 5 à 7, où la composante EA4 pourra être détectée soit (blocs 106, 108) par comparaison avec un gabarit, soit (blocs 110, 112) par recherche d'une énergie dans une fenêtre positionnée après les ondes EA1 et EA2 - qui existent, puisque dans cette situation le ventricule se contracte.

L'appareil pourra alors fonctionner en mode VDD standard (bloc 114), avec application d'un DAV sur détection des composantes EA4, et application d'un intervalle d'échappement IE en cas de non-détection de la composante EA4, typiquement en cas de bradycardie sinusale ou de tachycardie rapide auriculaire (flutter ou fibrillation auriculaire). En cas d'absence de détection de composante EA4 (bloc 116) sur un nombre Y prédéterminé de cycles, typiquement 20 cycles (bloc 118), alors le mode VDD est abandonné pour retourner en mode VVI avec rythme de base standard RB (bloc 120).

Si le mode VVI avec rythme de base réduit RBH (bloc 102) ne révèle aucune activité ventriculaire spontanée, on est alors vraisemblablement en présence d'un bloc AV complet (bloc de type III), c'est-à-dire d'une conduction atrioventriculaire déficiente où la dépolarisation des oreillettes n'est pas transmise aux ventricules, qui ne se contracteront pas spontanément (bloc 122).

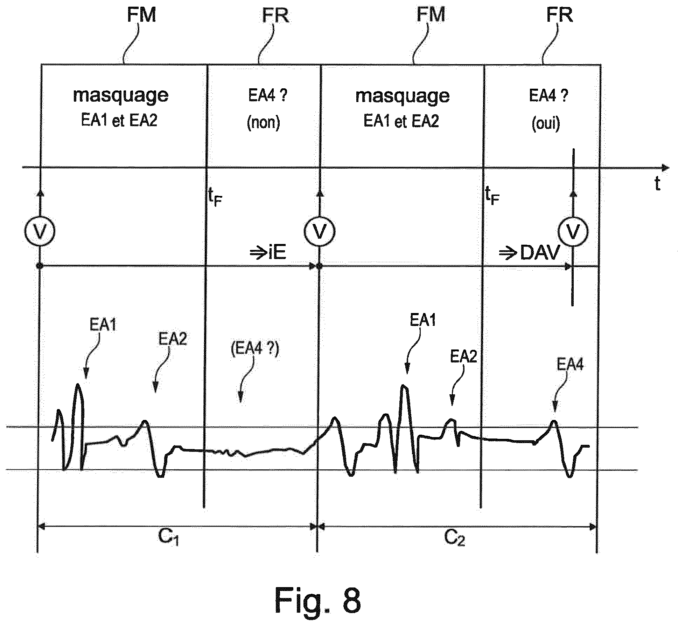

Dans la mesure où l'on ne dispose plus de marqueur d'activité électrique ventriculaire, une autre méthode, plus complexe, de recherche de la composante EA4 doit être utilisée, méthode qui sera décrite en référence aux Figures 8 à 11 : on attend alors qu'une onde EA4 entre dans une fenêtre de détection définie après les composantes EA1 et EA2 (bloc 124). Dès qu'une composante EA4 est trouvée (bloc 126), alors on peut retourner en mode VDD (bloc 114, décrit ci-dessus). En cas de non-détection de la composante EA4 sur un nombre Z prédéterminé de cycles, typiquement les 20 derniers cycles (bloc 128), alors on augmente légèrement la fréquence de stimulation, par exemple de + 5 bpm (bloc 130). En effet, il est possible que le rythme de base réduit RBH soit très proche du rythme du patient et qu'alors la composante EA2 ne tombe jamais dans sa fenêtre de recherche. Après ce changement de rythme, si aucune composante EA4 n'est trouvée (bloc 132) après un nombre T prédéterminé de cycles, typiquement 20 cycles (bloc 134) alors le mode VDD est abandonné pour retourner en mode VVI avec rythme de base standard RB (bloc 120, décrit ci-dessus).

### Cas des patients en rythme sinusal ou avec un bloc AV partiel (de type I ou II)

On va tout d'abord décrire, en référence aux Figures 5 à 7, une technique de détection de la composante EA4 applicable à des patients ne présentant qu'un bloc de branche partiel (de type I ou II).

Dans un tel cas, la conduction atrioventriculaire est préservée et il est possible de détecter une onde R et d'en dériver un marqueur d'activité électrique ventriculaire. D'autre part, le ventricule se contractant, le signal EA comprendra des composantes EA1 et EA2 liées à l'activité mécanique des ventricules.

Une fois acquis les signaux d'accélération EA et électriques EGM (étape 140), l'onde R est détectée dans le signal EGM (étape 142), de manière conventionnelle. Dans le signal EA, les composantes EA1 et EA2 sont détectées (étape 144), de manière à définir ensuite une fenêtre de recherche de la composante EA4 (étape 146), positionnée après la composante EA2.

Le dispositif recherche alors une composante EA dans la fenêtre ainsi définie (étape 148) :
- si une composante EA4 est présente (test 150), ceci révèle la présence d'une activité auriculaire effective, et il est alors possible d'appliquer un délai atrioventriculaire (DAV) de valeur préprogrammée (étape 152), compté à partir du marqueur d'activité auriculaire défini par la position temporelle de la composante EA4 détectée : on parvient alors au fonctionnement en mode VDD.
- en l'absence de composante EA4 dans la fenêtre de recherche, on peut supposer qu'il n'y a pas eu de contraction de l'oreillette. Dans ce cas, le dispositif applique un intervalle d'échappement (IE) prédéterminé (étape 154), c'est-à-dire un intervalle, compté à partir du dernier marqueur ventriculaire, à l'expiration duquel la stimulation ventriculaire sera automatiquement déclenchée (fonctionnement correspondant de fait à un mode VVI).

Dans le cas où un DAV est appliqué, celui-ci peut être établi de différentes manières :
- une valeur fixe prédéterminée ;
- une pluralité de valeurs fixes prédéterminées, par exemple une valeur différente à l'exercice et au repos, chacune de ces valeurs étant sélectionnée en fonction de l'état d'activité physique courant du patient ;
- une valeur ajustée dynamiquement en fonction du rythme cardiaque du patient (ce rythme étant déterminé à partir des intervalles entre marqueurs R détectés successifs) ; et/ou
- une valeur optimisée périodiquement en fonction d'indicateurs d'état clinique du patient, calculés à partir d'un historique des données d'accélération endocardiaque recueillies sur une période donnée et permettant d'évaluer l'évolution de l'état hémodynamique du patient.

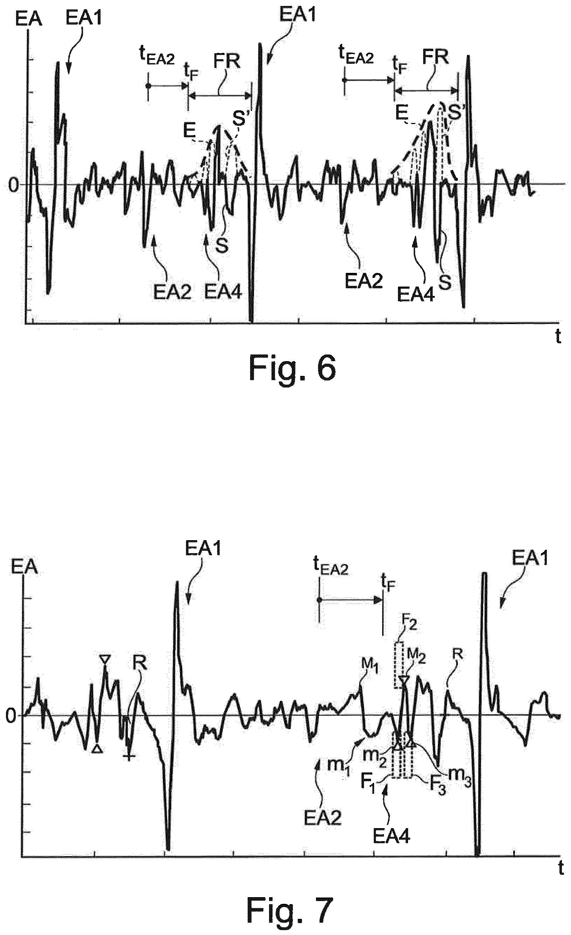

On va maintenant décrire, en référence aux Figures 6 et 7, deux manières possibles de détecter la présence d'une composante EA4 dans la fenêtre de recherche.

Avec la première technique, illustrée par la Figure 6 et correspondant aux blocs 110 et 112 de la Figure 4, le dispositif détecte tout d'abord la composante EA2 pour définir la position temporelle de la fenêtre de recherche de la composante EA4. La composante EA2 peut être détectée et marquée par divers procédés connus, tels que ceux décrits notamment dans le EP 2 092 885 A1 (ELA Medical) précité. L'analyse du signal EA permet ainsi de définir l'instant t_{EA2} de survenue de la composante EA2, ou, en variante, un instant correspondant à la fin de cette composante.

Une variante, en cas de difficulté à détecter la composante EA2, consiste à définir la position de la fenêtre de recherche en fonction de la composante EA1 ou de l'instant de détection de l'onde T.

Une fois la composante EA2 détectée, le dispositif calcule un instant t_{F} d'ouverture d'une fenêtre de recherche FR, le début de cette fenêtre FR étant calculé à partir de l'instant de survenue (ou de fin) de la composante EA2, instant auquel est ajouté un délai fixe ou bien fonction du rythme cardiaque instantané (tel que déterminé par la durée des intervalles RR successifs).

Dans la fenêtre de recherche FR, le signal S est redressé (signal S') et élevé au carré de manière à définir une enveloppe d'énergie E du signal EA redressé pendant la durée de la fenêtre FR. Après intégration, on obtient une valeur globale de l'énergie de la composante EA4 du signal, et cette valeur est comparée à un seuil prédéterminé : si le seuil est dépassé, on considère qu'une composante EA4 est effectivement présente, et inversement si le niveau d'énergie est en dessous du seuil on considère que l'on n'a pas détecté, ou pas pu détecter, de composante EA4.

La Figure 7 illustre une autre technique de détection de la composante EA4, basée sur une analyse morphologique et correspondant aux blocs 106 et 108 de la Figure 4. Cette technique est particulièrement adaptée au caractère pseudopériodique fortement marqué de la composante EA4.

Après avoir, comme dans le cas précédent, déterminé un marqueur temporel t_{EA2} lié à la détection de la composante EA2 et défini un instant t_{F} de début d'une fenêtre de recherche à partir de ce marqueur t_{EA2}, le signal EA est analysé pour rechercher les maxima locaux Mᵢ et minima locaux mᵢ situés dans la fenêtre de recherche, c'est-à-dire postérieurs à l'instant t_{F}. La détection de la composante EA4 consiste à déterminer si ces minima et maxima locaux se situent dans des fenêtres prédéterminées F₁, F₂, F₃... permettant d'appliquer des critères booléens pour décider de la détection ou non d'une composante EA4 : si par exemple deux des trois extrema locaux postérieurs au début de la fenêtre de recherche s'inscrivent dans les fenêtres prédéterminées, alors on considère qu'une composante EA4 est détectée.

Ainsi, sur l'exemple de la Figure 7 les extrema M₁ et m₁ ne sont pas pris en compte car antérieurs au début de la fenêtre de recherche. En revanche, les extrema suivants m₂, M₂ et m₃ sont pris en compte, et comme ils s'inscrivent tous les trois dans les fenêtres prédéterminées respectives F₁, F₂, F₃, alors il est possible de confirmer la détection d'une composante EA4.

Il est nécessaire de prévoir une phase d'apprentissage préalable pour définir la position des fenêtres d'analyse F₁, F₂, F₃... Cet apprentissage peut être effectué de la manière suivante :
- pour un nombre prédéterminé de battements, par exemple 30 battements, on détermine l'instant de la dépolarisation ventriculaire (marqueur R), qui servira de référence pour l'ouverture d'une fenêtre rétrospective ;
- tous les minima et maxima locaux du signal EA situés dans cette fenêtre rétrospective sont alors détectés ;
- sur le signal EA moyenné, on détecte les premiers, par exemple les trois ou quatre premiers, extrema locaux de la fenêtre de recherche (on choisit les premiers extrema locaux plutôt que les autres, en raison de leur relative stabilité d'un battement cardiaque à l'autre) ;
- à partir des ensembles d'extrema locaux respectifs ainsi enregistrés, on détermine la position temporelle et l'amplitude de chacune des fenêtres qui permettront de caractériser les positions relatives et les amplitudes typiques de la composante EA4.

### Cas des patients avec un bloc AV total (de type III)

On va maintenant décrire, en référence aux Figures 8 à 11, une technique de détection de la composante EA4 applicable à des patients présentant un bloc AV total (bloc AV de type III).

Dans une telle situation, la conduction atrioventriculaire est absente et le ventricule, qui ne se contracte pas spontanément, doit être stimulé.

Comme illustré Figure 8, il est nécessaire de définir une fenêtre de masquage FM permettant d'ignorer les composantes EA1 et EA2. La fenêtre de masquage FM est déclenchée sur le marqueur de stimulation ventriculaire V.

La détection de la composante EA4 est ensuite lancée, dans une fenêtre de recherche FR ouverte à partir d'un instant t_{F}. On notera qu'en pratique la contraction auriculaire peut être présente mais survenir de façon plus ou moins concomitante à la contraction (stimulée) du ventricule, de sorte qu'il peut arriver que la composante EA4 se superpose en fait aux composantes EA1 ou EA2 et que, bien que présente, ne soit pas détectée dans la fenêtre de recherche.

Selon qu'une composante EA4 est ou non détectée dans la fenêtre de recherche FR, l'action à prendre est différente :
- en l'absence de composante EA4 détectée (cas du cycle C1 illustré), le dispositif déclenche une stimulation ventriculaire après un intervalle d'échappement IE ;
- si une composante EA4 est détectée (cas du cycle C2 illustré) - ce qui signifie que la contraction auriculaire est effectivement présente, qu'elle peut être repérée dans le temps et qu'elle n'est pas concomitante à la contraction stimulée du ventricule -, alors on peut appliquer un DAV, compté à partir de l'instant de survenue (ou, en variante, de fin) de la composante EA4 détectée.

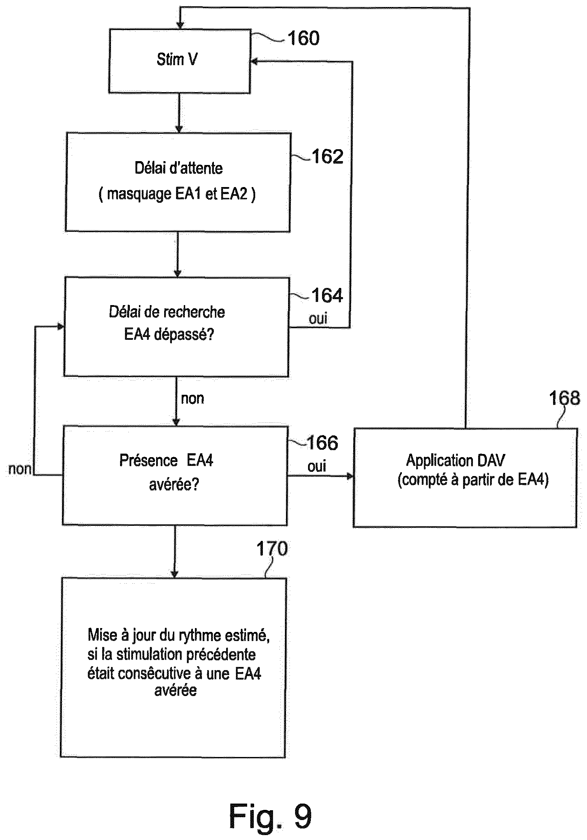

La Figure 9 est un organigramme résumant le mode opératoire appliqué par le procédé de l'invention, dans ce cas de bloc AV complet.

Après stimulation du ventricule (étape 160) un délai d'attente est compté (étape 162) de manière à masquer sur le signal EA les composantes EA1 et EA2 consécutives à la contraction du ventricule.

Après expiration de ce délai d'attente (correspondant à la fenêtre de masquage FM de la Figure 8), le signal EA est analysé pour y détecter la présence éventuelle d'une composante EA4 pendant la durée de la fenêtre de recherche FR (étape 164).

La présence d'une composante EA4 dans la fenêtre de recherche FR peut être mise en oeuvre par des techniques telles que celles décrites plus haut en référence aux Figures 6 et 7, à savoir par :
- détermination de l'énergie du signal EA dans une fenêtre prédéterminée, et seuillage ; et/ou
- comparaison du signal avec un gabarit, par exemple avec les extrema locaux du signal EA s'inscrivant dans des fenêtres prédéterminées.

À la fin de la fenêtre de recherche FR, le dispositif détermine la présence, ou non, d'une composante EA4 (étape 166) :
- dans l'affirmative, un DAV est appliqué (étape 168), compté à partir de la détection de la composante EA4, qui dans ce cas remplace le marqueur électrique auriculaire utilisé par les dispositifs mettant en oeuvre une électrode de détection dans l'oreillette ;
- dans la négative, à la fin de la fenêtre FR (étapes 170) une stimulation est systématiquement délivrée à l'issue d'un intervalle d'échappement IE.

Dans le cas où un DAV est appliqué, celui-ci peut être :
- une valeur fixe prédéterminée ;
- une pluralité de valeurs fixes prédéterminées, par exemple une valeur différente à l'exercice et au repos, chacune de ces valeurs étant sélectionnée en fonction de l'état d'activité physique courant du patient ;
- une valeur ajustée dynamiquement en fonction du rythme cardiaque du patient (ce rythme étant déterminé à partir des intervalles entre marqueurs R détectés successifs) ; et/ou
- une valeur optimisée périodiquement en fonction d'indicateurs d'état clinique du patient, calculés à partir d'un historique des données d'accélération endocardiaque recueillies sur une période donnée et permettant d'évaluer l'évolution de l'état hémodynamique du patient.

Dans le cas où un IE est appliqué, la durée de cet IE (c'est-à-dire la fréquence de stimulation en mode VVI) est avantageusement déterminée à partir d'une estimation du rythme spontané du patient, par exemple 80 % du rythme estimé ; ce rythme spontané est estimé à partir d'un certain nombre de battements cardiaques successifs, après moyennage des intervalles entre composantes EA4 successives. Le calcul de cet IE nécessite donc la détection des composantes EA4 de plusieurs cycles consécutifs pour en estimer l'intervalle, par exemple sur huit cycles. Si l'on n'a pas huit cycles consécutifs avec détection de la composante EA4, alors on applique l'intervalle d'échappement défini par le rythme de base réduit RBH (voir plus haut la description en référence à la Figure 4).

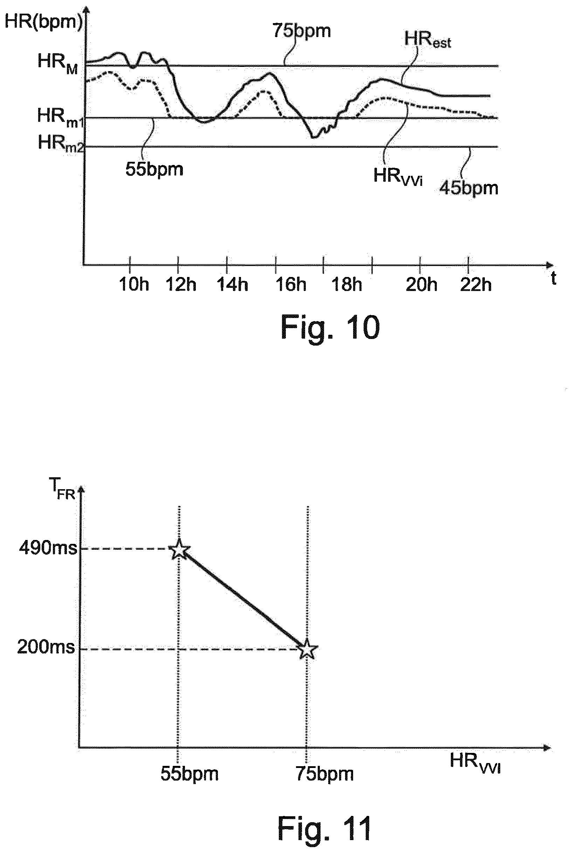

La Figure 10 illustre en HRₑₛₜ un tel exemple de rythme cardiaque estimé. Cette estimation est bornée par :
- une valeur maximale, définie par l'intervalle le plus court permettant une recherche satisfaisante de la composante EA4 par exemple : avec une fenêtre de masquage FM de 600 ms suivie d'une fenêtre de recherche FR de 200 ms (durée minimale de cette fenêtre), on obtient une durée de 600 + 200 = 800 ms, correspondant à un rythme cardiaque maximum HRM de 75 bpm, et
- une valeur minimale définissant le rythme en mode de stimulation VVI standard, par exemple avec une valeur HRₘ₁ pendant le jour de 55 bpm et HRₘ₂ pendant la nuit de 45 bpm. Ce rythme est le rythme de base réduit RBH décrit plus haut en référence à la Figure 4, qui peut éventuellement prendre deux valeurs différentes, une pour le jour et une pour la nuit.

On obtient ainsi une valeur estimée HR'ₑₛₜ du rythme cardiaque, à partir de laquelle est calculée la fréquence cardiaque à appliquer HR_{VVI} (par exemple HR_{VVI} = 80 % x HR'est et HR_{VVI} ≥ HRmin), qui définit une valeur correspondante de l'intervalle d'échappement IE.

La Figure 11 illustre la manière dont la durée T_{FR} de la fenêtre de recherche FR est adaptée en fonction du rythme cardiaque HR.

La valeur de rythme cardiaque utilisée est celle, HR_{VVI}, correspondant à l'intervalle d'échappement, déterminé comme expliqué ci-dessus à partir du rythme spontané et majoré ou minoré suivant les règles indiquées.

La durée de la fenêtre de recherche T_{FR} varie par exemple linéairement en fonction du rythme HR_{VVI}, entre une durée maximale de 490 ms pour un rythme cardiaque (minimal) de 55 bpm et une durée minimale de 200 ms pour un rythme cardiaque (maximal) de 75 bpm.

## Revendications

1. Un dispositif médical implantable actif (18) de type prothèse cardiaque de stimulation, resynchronisation et/ou défibrillation, comportant :
- un capteur d'accélération (26) apte à délivrer un signal d'accélération endocardiaque, EA, représentatif des contractions cycliques du myocarde ; et
- des moyens d'analyse du signal EA, aptes à reconnaitre et isoler dans le signal EA une composante EA4 correspondant au quatrième pic d'accélération endocardiaque associé à une activité auriculaire, **caractérisé en ce que** le dispositif est un dispositif dépourvu de moyens de recueil d'un signal EGM auriculaire,
**en ce qu'**il comprend en outre :
- des moyens de détection d'événements ventriculaires par analyse d'un signal EGM d'électrogramme ventriculaire ;
- des moyens de stimulation ventriculaire ;
- des moyens aptes à appliquer aux moyens de stimulation ventriculaire un délai atrioventriculaire, DAV, compté à partir d'un événement auriculaire et à l'issue duquel une stimulation ventriculaire (V) est délivrée en l'absence de détection d'un événement ventriculaire spontané (R) ;
- des moyens de détermination (146 ; 164) d'une fenêtre (FR) de recherche de la composante EA4 dans le signal EA ;
- des moyens aptes à avérer ou non (148 ; 166) la présence d'une composante EA4 dans ladite fenêtre de recherche (FR) ; et
- des moyens aptes à appliquer (142, 144 ; 160 ; 168) aux moyens de stimulation ventriculaire :
• ledit DAV, en présence d'une composante EA4 avérée ; ou
• un intervalle d'échappement prédéterminé, en l'absence de composante EA4 avérée,
et dans lequel les moyens de détermination de la fenêtre de recherche comprennent des moyens aptes :
a) en présence d'une conduction atrioventriculaire, à :
• détecter (142) l'instant de survenue de l'évènement ventriculaire (R) à partir du signal EGM ventriculaire ;
• détecter (144) dans le signal EA des composantes EA1 et/ou EA2 correspondant au premier et/ou second pics respectifs d'accélération endocardiaque associés à l'évènement ventriculaire ; et
• déterminer (146) la fenêtre (FR) de recherche de la composante EA4 en fonction de la position temporelle des composantes EA1 et/ou EA2 détectées, et
b) en l'absence de conduction atrioventriculaire, à :
• appliquer (162) une temporisation (FM) de masquage des composantes EA1 et/ou EA2 dans le signal EA, comptée à partir d'un événement ventriculaire stimulé (V) ; et
• déterminer (164) la fenêtre (FR) de recherche de la composante EA4 à l'issue de la temporisation de masquage.

2. Le dispositif de la revendication 1, dans lequel le dispositif est une capsule intracardiaque autonome (18) comprenant un corps tubulaire pourvu à son extrémité distale d'un organe d'ancrage (20) apte à pénétrer dans une paroi d'un ventricule d'un patient.

3. Le dispositif de la revendication 1, dans lequel les moyens aptes à avérer ou non la présence d'une composante EA4 dans la fenêtre de recherche (FR) comprennent des moyens aptes à évaluer le niveau d'énergie global (E) du signal EA dans la fenêtre de recherche (FR) et à comparer ce niveau d'énergie global à un seuil prédéterminé.

4. Le dispositif de la revendication 1, dans lequel les moyens aptes à avérer ou non la présence d'une composante EA4 dans la fenêtre de recherche (FR) comprennent des moyens d'analyse morphologique du signal EA dans la fenêtre de recherche (FR) par test du passage dans des fenêtres prédéterminées (F1, F2, F3) de minima locaux (m₁, m₂) et/ou de maxima locaux (M₁, M₂, M₃) du signal EA.

5. Le dispositif de la revendication 1, comprenant en outre des moyens d'estimation du rythme spontané (HRₑₛₜ) du patient en l'absence de conduction atrioventriculaire, calculé en fonction de l'intervalle de temps séparant la détection de deux composantes EA4 avérées pour des cycles cardiaques consécutifs.

6. Le dispositif de la revendication 5, comprenant des moyens aptes à calculer une fréquence de stimulation ventriculaire (HR_{VVI}) en fonction de la valeur de rythme spontané (HRₑₛₜ) calculée par les moyens d'estimation.

7. Le dispositif de la revendication 5, comprenant des moyens aptes à calculer l'intervalle d'échappement (IE) en fonction de la valeur de rythme spontané (HRₑₛₜ) calculée par les moyens d'estimation.

8. Le dispositif de la revendication 5, comprenant des moyens aptes à calculer le DAV en fonction de la valeur de rythme spontané (HRₑₛₜ) calculée par les moyens d'estimation.

9. Le dispositif de la revendication 5, comprenant des moyens aptes à calculer la durée (T_{FR}) de la fenêtre de recherche (FR) en fonction de la valeur de rythme spontané (HRₑₛₜ) calculée par les moyens d'estimation.

## Patentansprüche

1. Aktive implantierbare medizinische Vorrichtung (18) des Typs Stimulations-, Resynchronisations- und/oder Defibrillations-Herzprothese, umfassend:
- einen Beschleunigungssensor (26), der dazu geeignet ist, ein endokardiales Beschleunigungssignal EA auszugeben, das die zyklischen Kontraktionen des Herzmuskels repräsentiert ; und
- Mittel zur Analyse des EA-Signals, die dazu geeignet sind, in dem EA-Signal eine EA4-Komponente, die der vierten Spitze der endokardialen Beschleunigung entspricht, die der atrialen Aktivität zugeordnet ist, zu erkennen und zu isolieren,
**dadurch gekennzeichnet, dass** die Vorrichtung keine Mittel zum Detektieren eines atrialen EGM-Signals aufweist,
und dass sie außerdem Folgendes umfasst:
- Mittel zum Detektieren von ventrikulären Ereignissen durch Analyse eines Signals eines ventrikulären Elektrogramms (EGM);
- Mittel zur ventrikulären Stimulation;
- Mittel, die dazu geeignet sind, an den ventrikulären Stimulationsmitteln eine atrioventrikuläre Verzögerung, DAV, anzuwenden, die ab einem atrialen Ereignis gezählt wird und nach der in Abwesenheit der Detektion eines spontanen ventrikulären Ereignisses (R) eine ventrikuläre Stimulation (V) angewendet wird;
- Mittel zur Bestimmung (146; 164) eines Fensters (FR) zur Suche der EA4-Komponente im EA-Signal;
- Mittel (148; 166), die dazu geeignet sind, die Anwesenheit einer EA4-Komponente im Suchfenster (FR) zu bestätigen oder zu widerlegen;
- Mittel (142, 144; 160; 168), die dazu geeignet sind, an den ventrikulären Stimulationsmitteln:
• bei bestätigter Anwesenheit einer EA4- Komponente, die DAV anzuwenden; oder
• bei bestätigter Abwesenheit einer EA4-Komponente ein vorbestimmtes Ersatzintervall anzuwenden,
und bei der die Mittel zur Bestimmung des Suchfensters Mittel umfassen, die dazu geeignet sind:
a) in Anwesenheit einer atrioventrikulären Leitung:
• basierend auf dem ventrikulären EGM-Signal, den Zeitpunkt des Auftretens eines ventrikulären Ereignisses (R) zu detektieren (142);
• in dem EA-Signal, EA1- und/oder EA2-Komponenten zu detektieren (144), die der jeweils ersten und/oder zweiten Spitze der endokardialen Beschleunigung entsprechen, die dem ventrikulären Ereignis zugeordnet sind,
• in Abhängigkeit der zeitlichen Position der erfassten EA1- und/oder EA2-Komponenten, das Fenster (FR) zur Suche der EA4-Komponente zu bestimmen (146), und
b) in Abwesenheit einer atrioventrikulären Leitung:
• eine Verzögerung (FM) zur Maskierung der EA1- und/oder EA2-Komponenten im EA-Signal anzuwenden (162), die ab einem stimulierten ventrikulären Ereignis (V) gezählt wird; und
• nach der Verzögerung zur Maskierung, das Fenster (FR) zur Suche der EA4-Komponente zu bestimmen (164).

2. Vorrichtung nach Anspruch 1, bei der die Vorrichtung eine autonome intrakardiale Kapsel (18) ist, die einen rohrförmigen Körper aufweist, der an seinem distalen Ende mit einem Verankerungselement (20) versehen ist, das dazu geeignet ist, in die Wand eines Ventrikels eines Patienten einzudringen.

3. Vorrichtung nach Anspruch 1, bei der die Mittel zur Bestätigung oder Widerlegung der Anwesenheit einer EA4-Komponente im Suchfenster (FR) Mittel aufweisen, die dazu geeignet sind, ein Gesamtenergieniveau (E) des EA-Signals im Suchfenster (FR) zu schätzen und dieses Gesamtenergieniveau mit einem vorbestimmten Schwellwert zu vergleichen.

4. Vorrichtung nach Anspruch 1, bei der die Mittel zur Bestätigung oder Widerlegung der Anwesenheit einer EA4-Komponente im Suchfenster (FR) Mittel zur morphologischen Analyse des EA-Signals im Suchfenster (FR) aufweisen, die den Durchlauf von lokalen Minima (m₁, m₂) und/oder lokalen Maxima (M₁, M₂, M₃) des EA-Singals in vorbestimmten Fenstern (Fl, F2, F3) prüfen.

5. Vorrichtung nach Anspruch 1, weiter umfassend Mittel zur Schätzung des spontanen Rhythmus (HRₑₛₜ) des Patienten in Abwesenheit atrioventrikulärer Leitung, welcher in Abhängigkeit des Zeitintervalls zwischen der Erfassung zweier bestätigter EA4-Komponenten in aufeinanderfolgenden Herzzyklen berechnet wird.

6. Vorrichtung nach Anspruch 5, umfassend Mittel, die dazu geeignet sind, eine Frequenz der ventrikulären Stimulation (HR_{VVI}) in Abhängigkeit des durch die Schätzungsmittel berechneten Werts des spontanen Rhythmus (HRₑₛₜ) zu berechnen.

7. Vorrichtung nach Anspruch 5, umfassend Mittel, die dazu geeignet sind, das Ersatzintervall (IE) in Abhängigkeit des durch die Schätzungsmittel berechneten Werts des spontanen Rhythmus (HRₑₛₜ) zu berechnen.

8. Vorrichtung nach Anspruch 5, umfassend Mittel, die dazu geeignet sind, die DAV in Abhängigkeit des durch die Schätzungsmittel berechneten Werts des spontanen Rhythmus (HRₑₛₜ) zu berechnen.

9. Vorrichtung nach Anspruch 5, umfassend Mittel, die dazu geeignet sind, die Dauer (T_{FR}) des Suchfensters (FR) in Abhängigkeit des durch die Schätzungsmittel berechneten Werts des spontanen Rhythmus (HRₑₛₜ) zu berechnen.

## Claims

1. An active implantable medical device (18) of the cardiac prosthesis type for stimulation, resynchronization and/or defibrillation, comprising:
- an acceleration sensor (26) adapted to deliver an endocardial acceleration (EA) signal, representative of the cyclic contractions of the myocardium; and
- means for analyzing the EA signal, adapted to recognize and isolate in the EA signal an EA4 component corresponding to the fourth peak of endocardial acceleration associated with atrial activity,
**characterized in that** the device is a device lacking means for collecting an atrial EGM signal,
and **in that** it further comprises:
- means for detecting ventricular events by analysis of a ventricular electrogram (EGM) signal;
- means for ventricular stimulation;
- means adapted to apply, to the means for ventricular stimulation, an atrioventricular delay (AVD) counted from an atrial event and at the end of which a ventricular stimulation (V) is delivered in the absence of detection of a spontaneous ventricular event (R); and
- means for determining (146; 164) a search window (FR) of the EA4 component in the EA signal;
- means adapted to confirm the presence or absence of an EA4 component in the search window (FR); and
- means adapted to apply (142; 144; 160; 168) to the ventricular stimulation means:
• the AVD, in the presence of a confirmed EA4 component; or
• a predetermined escape interval in the absence of a confirmed EA4 component,
and wherein the means for determining the search window comprises means adapted to:
a) in the presence of atrioventricular conduction:
• detect (142) the time of occurrence of a ventricular event (R) from the ventricular EGM signal;
• detect (144) in the EA signal EA1 and/or EA2 components corresponding to the respective first and/or second peaks of endocardial acceleration associated with the ventricular event; and
• determine (146) the search window (FR) of the EA4 component depending on the temporal position of the detected EA1 and/or EA2 components, and
b) in the absence of atrioventricular conduction:
• apply (162) a masking delay (FM) of the EA1 and/or EA2 components in the EA signal, counted from a stimulated ventricular event (V); and
• determine (164) the search window (FR) of the EA4 component after the masking delay.

2. The device according to claim 1, wherein the device is an intracardiac autonomous capsule (18) comprising a tubular body provided at its distal end with an anchoring member (20) capable of penetrating into a wall of a ventricle of a patient.

3. The device according to claim 1, wherein the means adapted to confirm the presence or absence of the EA4 component in the search window (FR) comprises means adapted to assess the overall energy level (E) of the signal EA in the search window (FR) and comparing the overall energy level to a predetermined threshold.

4. The device according to claim 1, wherein the means adapted to confirm the presence or absence of the EA4 component in the search window (FR) comprises means for morphological analysis of the EA signal in the search window (FR) by testing the passing through predetermined windows (Fl, F2, F3) of local minima (m₁, m₂) and/or of local maxima (M₁, M₂, M₃) of the EA signal.

5. The device according to claim 1, further comprising means for estimating the spontaneous rhythm (HRₑₛₜ) of the patient in the absence of atrioventricular conduction, calculated based on the time interval separating the detection of two confirmed EA4 components for consecutive cardiac cycles.

6. The device according to claim 5, comprising means adapted to calculate a ventricular stimulation rate (HR_{VVI}) depending on the value of spontaneous rhythm (HRₑₛₜ) calculated by the estimation means.

7. The device according to claim 5, comprising means adapted to calculate the escape interval (IE) according to the value of spontaneous rhythm (HRₑₛₜ) calculated by the estimation means.

8. The device according to claim 5, comprising means adapted to calculate the AVD in accordance with the value of spontaneous rhythm (HRₑₛₜ) calculated by the estimation means.

9. The device according to claim 5, comprising means adapted to calculate the duration (T_{FR}) of the search window (FR) based on the value of spontaneous rhythm (HRₑₛₜ) calculated by the estimation means.
